# EUROPÄISCHE PATENTANMELDUNG

(11) **EP 0 527 385 A1**
(43) Veröffentlichungstag der Anmeldung: **17.02.1993**
(21) Anmeldenummer: 92112847.6
(22) Anmeldetag: 28.07.1992
(51) Int. Cl.: C07D 217/14, C07D 215/12, C07D 209/10

(54) **Alpha-Trifluormethyl-substituierte, gesättigt-bicyclische Amine und Verfahren zu deren Herstellung**

(30) Priorität: 10.08.1991 DE 4126482
(71) Anmelder: BAYER AG, 51368 Leverkusen (DE)
(72) Erfinder: Negele, Michael, Dr., W-5650 Solingen 11 (DE); Häbich, Dieter, Dr., W-5600 Wuppertal 1 (DE); Laue, Christian, Dr., W-4018 Langenfeld (DE); Ziemann, Heinz, Dr., W-5653 Leichlingen (DE)

(57) **Zusammenfassung**

Neue α-trifluormethyl-substituierte, gesättigt-bicyclische Amine der Formel 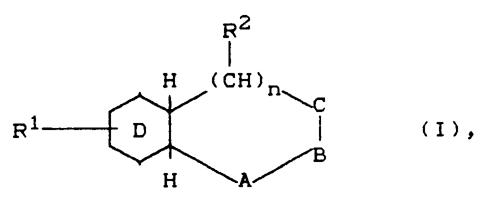
in der
- R¹: für Wasserstoff, C₁- bis C₈-Alkyl oder C₁- bis C₈-Alkoxy,
- R²: für Wasserstoff oder C₁- bis C₄-Alkyl,
- n: für Null oder 1,
- A: für 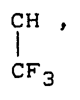 NH oder 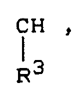
- B: für NH oder 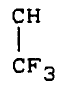 und
- C: für 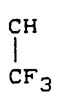 oder 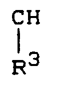 stehen,
wobei A, B und C entweder jeweils verschieden voneinander sind oder C für 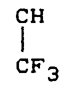 und eines von A
und B für NH und das andere für 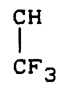 steht
und R³ Wasserstoff oder C₁- bis C₄-Alkyl bedeutet und
der Ring D gesättigt ist.

## Beschreibung

Die vorliegende Erfindung betrifft neue α-trifluormethyl-substituierte, gesättigt-bicyclische Amine und ein Verfahren zu deren Herstellung.

In der US-PS 3 959 333 wird beschrieben, daß man α-Trifluormethyl-1,2,3,4-tetrahydroisochinoline durch katalytische Hydrierung von α-Trifluormethyl-3,4-dihydroisochinolinen an Platinoxid-Katalysatoren herstellen kann. Perhydrierte (= gesättigte) Produkte werden dabei nicht erhalten.

In J. Chem. Soc., Perkin Trans. II, S. 615 (1972) ist beschrieben, daß sich nicht Trifluormethyl-substituiertes Chinolin im Verlauf von 9 Tagen an Platin-Schwarz-Katalysatoren zu gesättigtem Dekahydrochinolin hydrieren läßt.

Es wurden nun α-trifluormethyl-substituierte, gesättigtbicyclische Amine der Formel (I) gefunden 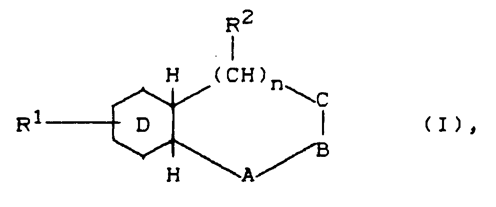
in der
- R¹: für Wasserstoff, C₁- bis C₈-Alkyl oder C₁- bis C₈-Alkoxy,
- R²: für Wasserstoff oder C₁- bis C₄-Alkyl,
- n: für Null oder 1,
- A: für 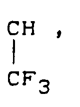 NH oder 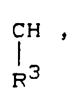
- B: für NH oder 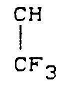 und
- C: für 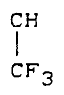 oder 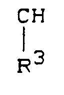 stehen,
wobei A, B und C entweder jeweils verschieden voneinander sind oder C für 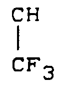 und eines von A
und B für NH und das andere für 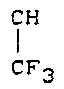 steht
und R³ Wasserstoff oder C₁- bis C₄-Alkyl bedeutet und
der Ring D gesättigt ist.

Bevorzugt sind Verbindungen der Formel (I), bei denen
- R¹: für Wasserstoff, Methyl oder Methoxy,
- R²: für Wasserstoff und/oder
- R³: für Wasserstoff stehen und
n, A, B, C und D die oben angegebene Bedeutung haben.

Die vorliegende Erfindung betrifft weiterhin ein Verfahren zur Herstellung der α-trifluormethylsubstituierten, gesättigt-bicyclischen Amine der Formel (I), das dadurch gekennzeichnet ist, daß man entsprechende ungesättigte Verbindungen mit molekularem Wasserstoff in Gegenwart eines Ruthenium-Katalysators hydriert.

Bei den entsprechenden ungesättigten Verbindungen kann es sich um nicht- oder teilhydrierte Chinoline, Isochinoline und Indole handeln, die α-ständig zum Stickstoffatom einen Trifluormethyl-Substituenten, am Stickstoff enthaltenen Ring gegebenenfalls 1 bis 2 C₁-bis C₄-Alkyl-Substituenten und am carbocyclischen Ring gegebenenfalls einen C₁- bis C₈-Alkyl- oder C₁- bis C₈-Alkoxy-Substituenten enthalten. Derartige Chinoline, Isochinoline und Indole sind bekannt oder analog den bekannten herstellbar (siehe z.B. J. Org. Chem. 27, 1406 (1962) und US-PS 3 956 333). Vorzugsweise kommen teilhydrierte Chinoline und Isochinoline der beschriebenen Art zum Einsatz.

Die erfindungsgemäße Hydrierung kann beispielsweise bei Temperaturen von 20 bis 150°C und Drucken im Bereich von 60 bis 180 bar durchgeführt werden. Bevorzugt sind Temperaturen von 70 bis 110°C und Drucke von 80 bis 120 bar. Im allgemeinen ist es günstig, die Hydrierung in Gegenwart eines Lösungsmittels durchzuführen. Als Lösungsmittel kommen beispielsweise Alkohole, Ether, gesättigte Kohlenwasserstoffe und Wasser in Frage. Bevorzugt sind Ethanol, Tetrahydrofuran und Cyclohexan.

Bei den erfindungsgemäß einzusetzenden Ruthenium-Katalysatoren kann es sich um beliebige, aus Ruthenium und/oder Rutheniumverbindungen bestehende oder Ruthenium und/oder Rutheniumverbindungen enthaltende Katalysatoren handeln. Vorzugsweise kommen Trägerkatalysatoren zum Einsatz, die beispielsweise 0,1 bis 20 Gew.-% Ruthenium und/oder Rutheniumverbindungen enthalten. Geeignete Trägermaterialien sind beispielsweise Aluminiumoxid, Siliciumoxid, Bariumsulfat, Titandioxid und Kohlen der verschiedensten Art. Besonders bevorzugt sind Katalysatoren, die Ruthenium und/oder Rutheniumdioxid auf einem Aluminiumoxidträger enthalten.

Es ist überraschend, daß beim erfindungsgemäßen Verfahren aus dem vorhandenen CF₃-Substituenten nicht oder nicht in nennenswertem Umfang Fluorwasserstoff abgespalten wird und minderfluorierte oder unfluorierte Produkte entstehen, denn aus Fluorine Chemistry Reviews 1, 315-358 (1967), insbesondere Seiten 323-324, ist bekannt, daß Haloalkyl-Gruppen in Gegenwart von Wasserstoff nicht immer stabil sind.

Erfindungsgemäß kann man Verbindungen der Formel (I) mit guten Ausbeuten, in hohen Reinheiten und in kurzen Reaktionszeiten erhalten.

Normalerweise fallen die Verbindungen der Formel (I) als Diastereomerengemisch an. Wenn bei den in das erfindungsgemäße Verfahren eingesetzten nicht- oder teilhydrierten Chinolinen, Isochinolinen und Indolen mit einem Trifluormethyl-Substituenten α-ständig zum Stickstoffatom eine enantiomere Form angereichert ist, so werden im allgemeinen Verbindungen der Formel (I) erhalten, bei denen dann eine diastereomere Form angereichert ist. Bevorzugt findet eine cis-Hydrierung statt.

Enantiomer angereichertes 1-Trifluormethyl-1,2,3,4-tetrahydroisochinolin kann man in an sich bekannter Weise, z.B. durch Hydrierung von 1-Trifluormethyl-3,4-dihydro-isochinolin mit
a) Wasserstoff und chiralen Katalysatoren (z.B. dem in-situ-Katalysator [Rh(nbd)Cl]₂ / chirales Phosphin, z.B. Prophos, Dipamp oder Diop - (siehe z.B. EP-OS 0 302 021 - nbd bedeutet Norbornadien, Prophos bedeutet 2,3-Diphenylphosphinopropan, Dipamp bedeutet 1,2-Di-(o-tolyl-phenyl)-phosphinoethan und Diop bedeutet 2,2-Dimethyl-4,5-diphenylphosphino-1,3-dioxolan) oder
b) Silanen als Wasserstoffquelle (z.B. Diphenylsilan und dem in-situ-Katalysator [Rh(cod)Cl]₂ / (-)Diop - (siehe Angew. Chem. Int. Ed. Engl. 24, 995 (1985) - cod bedeutet 1,5-Cyclooctadien, Diop siehe oben) oder
c) chiralen Reduktionsreagenzien (z.B. mit Tris-[(S)-N-Benzyloxycarbonyl-propyloxy]-hydroborat - siehe J.C.S. Perkin Trans. 1, 1983, 265) herstellen.

Die erfindungsgemäßen α-trifluormethyl-substituierten, gesättigt-bicyclischen Amine der Formel (I) sind wertvolle Zwischenprodukte zur Herstellung von retroviralen Mitteln. Beispielsweise kann man ein Amin der Formel (I) in einem der Verfahren gemäß den folgenden Formelschemata [A] bis [D] anstelle des dort angegebenen α-trifluormethylsubstituierten Pyrrolidins bzw. α-trifluormethylsubstituierten Piperidins einsetzen. Man erhält so retrovirale Mittel, die jeweils der letzten Formel der Verfahrensschemata entsprechen, die jedoch anstelle eines α-Trifluormethylpyrrolidin- bzw. α-Trifluormethylpiperidin-Ringes einen Bicyclus enthalten, der dem jeweils eingesetzten Amin der Formel(I) entspricht. Die in den Formelschemata [A] bis [D] gemachten Angaben zu Einsatzprodukten, Endprodukten, Lösungsmitteln, Hilfsmitteln etc. sind dabei nur beispielhaft und können im Rahmen des fachmännischen Wissens variiert werden.

Derartige retrovivale Mittel sind Gegenstand einer gleichzeitig eingereichten weiteren Patentanmeldung des gleichen Anmelders.

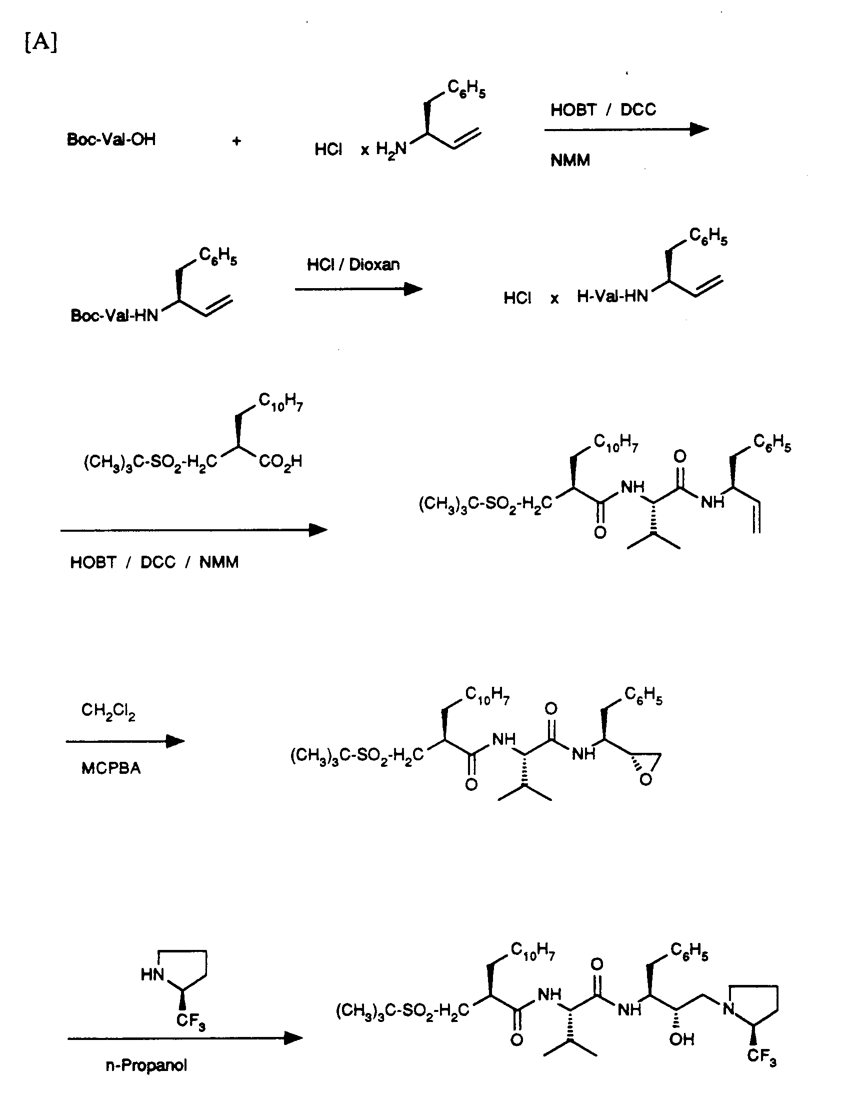

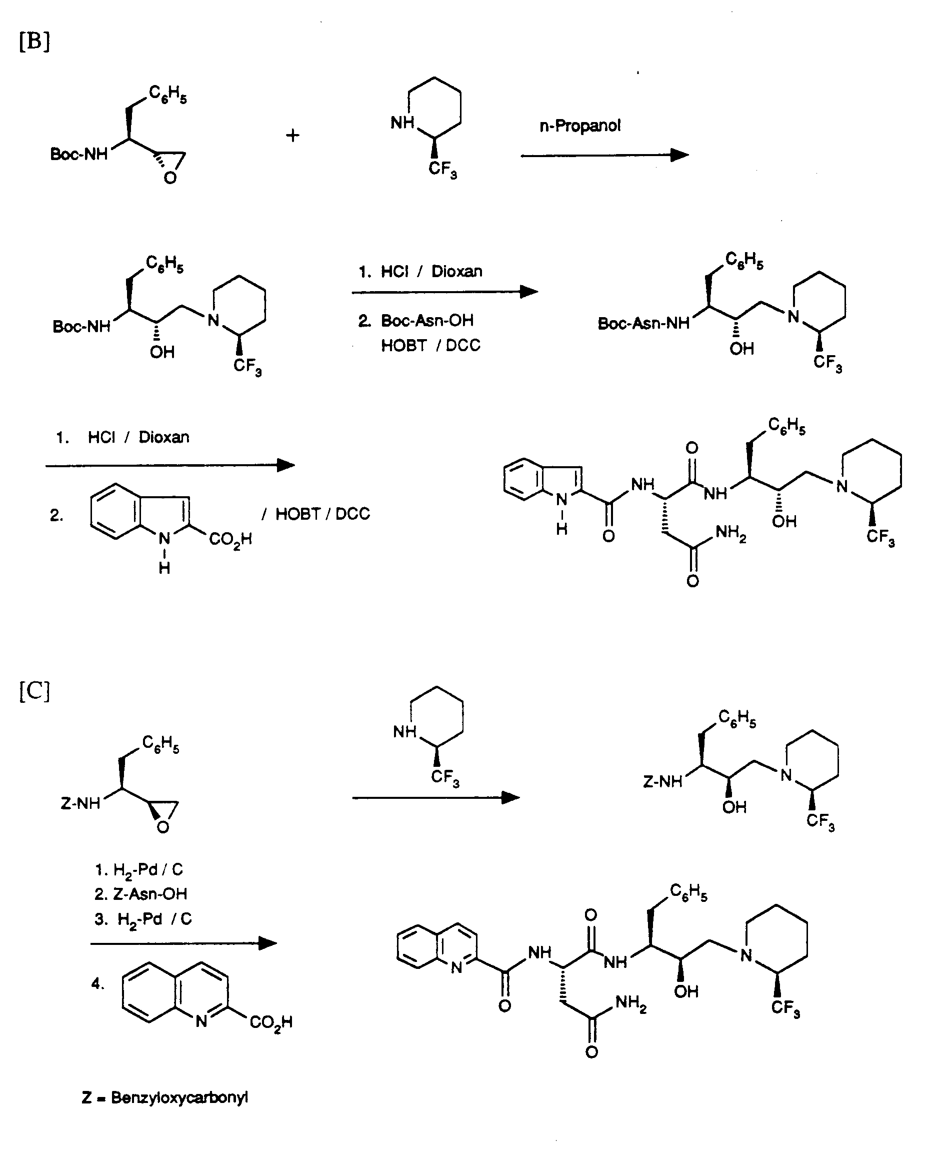

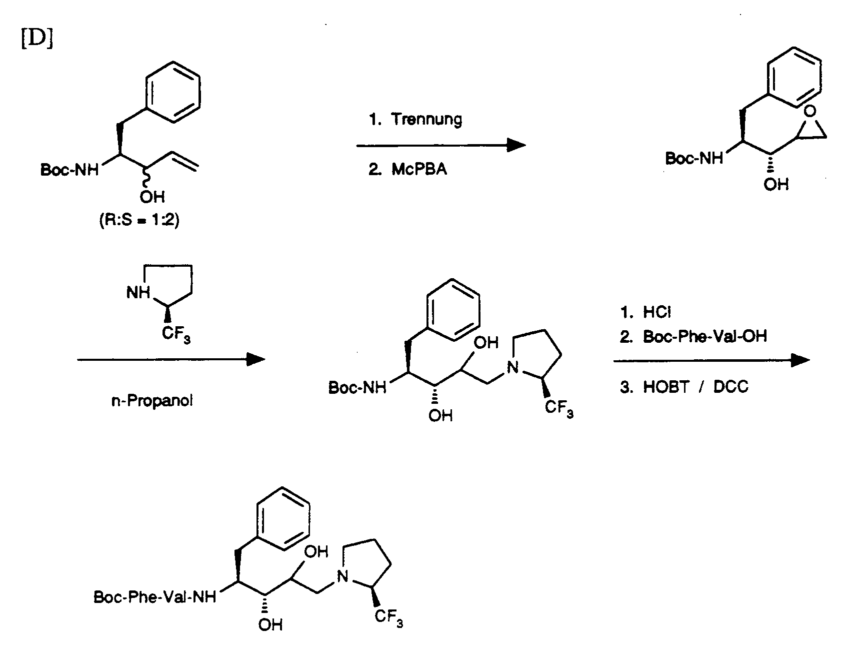

### Beispiele

### Beispiel 1

2-(Trifluormethyl)-decahydrochinolin (Formel (I); R¹ = H, R² = H, n = 1, A = NH, B = CH-CF₃ und C = CH-R³ mit R³ = H).

15 g (0,076 mol) 2-(Trifluormethyl)-chinolin [hergestellt aus Chinolin-2-carbonsäure nach J. Org. Chem. 27, 1406 (1962)] wurden in 100 ml Tetrahydrofuran an 2,5 g eines Katalysators enthaltend 10 Gew.-% Ruthenium auf Aluminiumoxid bei 180°C 8 Std. lang bei 80 bis 100 bar Wasserstoffdruck in einem 0,3 l Edelstahl-Autoklaven hydriert. Nach Filtration wurde das Tetrahydrofuran abgezogen und das Rohprodukt im Wasserstrahlvakuum über eine Mikrodestillationsapparatur fraktioniert.

Kp₁₆ (Hauptfraktion): 88°C.
Ausbeute: 12,5 g (= 0,061 mol = 80 % der Theorie). Das Produkt fiel als Diastereomeren-Gemisch an.
- ¹⁹F-NMR:: δ = +0,65 ppm (d, J_{H-F} = 7,1 Hz) (lt. Integral ca; 88 %);
- ¹⁹F-NMR:: δ = +0,90 ppm (d, J_{H-F} = 7,1 Hz) (lt. Integral ca. 12 %) (gegen CF₃COOH als externen Standard).

### Beispiel 2

1-(Trifluormethyl)-decahydroisochinolin (Formel (I); R¹ = H, R² = H, n = 1, A = CH-CF₃, B = NH, und C = CH-R³ mit R³ = H).

20 g (0,101 mol) 1-(Trifluormethyl)-3,4-dihydroisochinolin [hergestellt aus N-(Phenethyl)-trifluoracetamid analog US-PS 3 956 333] wurden in 100 ml Tetrahydrofuran an 5 g eines Katalysators enthaltend 10 Gew.-% Ruthenium auf Aluminiumoxid bei 150°C 8 Std. lang bei 100 bar Wasserstoffdruck in einem 0,3 l Edelstahl-Autoklaven hydriert. Nach Filtration wurde das Tetrahydrofuran abgezogen und das Rohprodukt im Wasserstrahlvakuum über eine Mikrodestillationsapparatur fraktioniert.

Kp₁₆ (Hauptfraktion); 94 bis 96°C.
Ausbeute: 19 g (= 0,092 mol = 91 % der Theorie).
Das Produkt fiel als Diastereomeren-Gemisch an.
- ¹⁹F-NMR:: δ = +5,9 ppm (d, J_{H-F} = 8,5 Hz) (lt. Integral ca: 99 %);
- ¹⁹F-NMR:: δ = +14,2 ppm (d, J_{H-F} = 8,5 Hz) (lt. Integral < 1 %) (gegen CF₃COOH als externen Standard).

### Beispiel 3

3-(Trifluormethyl)-decahydroisochinolin (Formel (I); R¹ = H, R² = H, n = 1, A = CH-R³ mit R³ = H, B = NH und C = CH-CF₃).

20 g (0,101 mol) 3-(Trifluormethyl)-3,4-dihydroisochinolin [hergestellt aus N-(1,1,1-Trifluor-3-phenyl-2-propyl)-formamid analog US-PS 3 956 333] wurden in 100 ml Tetrahydrofuran an 5 g Katalysator enthaltend 10 Gew.-% Ruthenium auf Aluminiumoxid bei 150 bis 180°C 8 Std. lang bei 90 bis 110 bar Wasserstoffdruck in einem 0,3 l Edelstahl-Autoklaven hydriert. Nach Filtration wurde das Tetrahydrofuran abgezogen und das Rohprodukt im Wasserstrahlvakuum über eine Mikrodestillationsapparatur fraktioniert.

Hauptfraktion; Kp₁₆ 93 bis 94°C.
Ausbeute: 18,5 g (= 0,090 mol = 88 % der Theorie).
Das Produkt fiel als Diastereomerengemisch an.
- ¹⁹F-NMR:: δ = -1,1 ppm (d, J_{H-F} = 7 Hz) (lt. Integral ca: 94 %);
- ¹⁹F-NMR:: δ = -1,3 ppm (d, J_{H-F} = 7 Hz) (lt. Integral ca. 6 %) (gegen CF₃COOH als externen Standard).

### Beispiel 4

2-(Trifluormethyl-)-octahydroindol (Formel (I); R¹ = H, n = Null, A = NH, B = CH-CF₃ und C = CH-R³ mit R³ = H).

5 g (0,025 mol) 2-(Trifluormethyl-)-indol [hergestellt aus 2-(Trifluormethyl)-chinolin (vgl. Beispiel 1) nach Y. Kobayashi, J. Org. Chem. 39, 1836 (1974)] wurden in 70 ml Tetrahydrofuran an 2 g Ruthenium auf Aluminiumoxid (10 Gew.-% Ru-Gehalt) bei 180°C 6 Stunden lang bei 70 bis 90 bar Wasserstoffdruck in einem 0,3 l Edelstahl-Autoklaven hydriert.

Nach Filtration wurde das Tetrahydrofuran abgezogen und das Rohprodukt im Wasserstrahlvakuum über eine Mikrodestillationsapparatur fraktioniert.

Hauptfraktion: Kp₁₆: 72 bis 75°C; Ausbeute 4.1 g (0,022 mol= 88 % der Theorie).
Das Produkt fiel als Diastereomerengemisch an.
- ¹⁹F-NMR:: δ = -1,3 ppm (d, J_{H-F} = 7 Hz) (lt. Integral ca: 78 %);
- ¹⁹F-NMR:: δ = -1,7 ppm (d, J_{H-F} = 7Hz) (lt. Integral ca. 22 %) (gegen CF₃COOH als externen Standard).

### Beispiel 5

1-Methyl-3-(trifluormethyl)-nonahydroisochinolin (Formel (I); R¹ = H, R² = H, n = 1, A = CH-R³ mit R³ = H, B = NH und c = CH-CF₃).

20 g (0,094 mol) 1-Methyl-3-(trifluormethyl)-3,4-dihydroisochinolin [hergestellt aus N-(1,1,1-Trifluor-3-phenyl-2-propyl)-acetamid analog US-Pat. 3 956 333] wurden in 100 ml Tetrahydrofuran an 5 g Ruthenium auf Aluminiumoxid (10 Gew.-%) Ru-Gehalt) bei 140 bis 160°C 10 Stunden lang bei 80 bis 100 bar Wasserstoffdruck in einem 0,3 l Edelstahl-Autoklaven hydriert.

Nach Filtration wurde das Tetrahydrofuran abgezogen und das Rohprodukt im Wasserstrahlvakuum über eine Mikrodestillationsapparatur fraktioniert.

Hauptfraktion; Kp₁₆: 102 bis 106°C; Ausbeute 17,8 g (0,081 mol = 86,4 % der Theorie).
Das Produkt fiel als Diastereomerengemisch an.
- ¹⁹F-NMR:: δ = -1,8 ppm (d, J_{H-F} = 7 Hz) (lt. Integral ca: 82 %);
- ¹⁹F-NMR:: δ = -2,1 ppm (d, J_{H-F} = 7 Hz) (lt. Integral ca. 18 %) (gegen CF₃COOH als externen Standard).

### Beispiel 6

1,3-Bis-(trifluormethyl)-nonahydroisochinolin (Formel (I); R¹ = H, R² = H, n = 1, A = CH-CF₃, B = NH und C = CH-CF₃).

20 g (0,074 mol) 1,3-Bis-(trifluormethyl)-3,4-dihydroisochinolin (hergestellt aus N-(1,1,1-Trifluor-3-phenyl-2-propyl)-trifluoracetamid analog US-Pat. 3 956 333] wurden in 100 ml Tetrahydrofuran an 5 g Ruthenium auf Aluminiumoxid (10 Gew.-%) Ru-Gehalt) bei 150 bis 180°C 8 Stunden lang bei 100 bis 120 bar Wasserstoffdruck in einem 0,3 l Edelstahl-Autoklaven hydriert.

Nach Filtration wurde das Tetrahydrofuran abgezogen und das Rohprodukt im Wasserstrahlvakuum über eine Mikrodestillationsapparatur fraktioniert.

Hauptfraktion; Kp₁₆: 108 bis 112°C; Ausbeute 15,4 g (0,056 mol = 76 % der Theorie).
Das Produkt fiel als Diastereomerengemisch an,
- ¹⁹F-NMR:: δ = -1,9 ppm (d, J_{H-F} = 7,5 Hz)
δ = +5,6 ppm (d, J_{H-F} = 7 Hz) (lt. Integral ca: 95 %);
δ = -2,8 ppm (d, J_{H-F} = 7,5 Hz)
δ = +11,3 ppm (d, J_{H-F} = 7 Hz) (lt. Integral ca: 5 %);
(gegen CF₃COOH als externen Standard).

### Beispiel 7

### Enantiomer angereichertes (1)-Trifluormethyl-1,2,3,4-tetrahydroisochinolin

Eine Lösung aus 467 mg (2,3 mmol) (1)-Trifluormethyl-3,4-dihydroisochinolin und 7,4 g (9,5 mmol) Tris-[(S)-N-Benzyloxycarbonylpropyloxy]-hydroborat in 20 ml Methylenchlorid wurden 36 Std. bei 20°C gerührt. Das Gemisch wurde mit 3 molarer Oxalsäure auf pH 3 eingestellt und 15 min gerührt. Anschließend wurde mit festem Kaliumcarbonat alkalisch gestellt und mit Essigsäureethylester extrahiert. Die organische Phase wurde mit Wasser und gesättigter Natriumchloridlösung extrahiert, mit Magnesiumsulfat getrocknet und eingeengt. Nach Destillation am Kugelrohr (110°C, 0,15 mbar) blieben 430 mg (91 % der Theorie) eines farblosen Öls (GC 95 %) zurück.

Zur Bestimmung des Enantiomerenüberschusses wurde die Substanz in den "Mosher-Ester" überführt:
Eine Losung aus 10 mg (0,05 mmol) (1)-Trifluormethyl-1,2,3,4-tetrahydro-isochinolin, 38 mg (+)-Methoxy-phenyltrifluormethyl-essigsäurechlorid und 1 mg Dimethylaminopyridin in 1 ml Methylenchlorid wurden 24 Std. bei 20°C gerührt und anschließend mit Wasser 15 min lang hydrolysiert. Nach Extraktion nacheinander mit 1N Salzsäure, 1N Natronlauge, Wasser und gesättigter Natriumchloridlösung und anschließender Trocknung mit Magnesiumsulfat wurde gaschromatographisch analysiert. (20 m SE30; 30 ml He/min, 150 bis 340°C, 10°C/min).

Eluationszeiten der beiden Diastereomere; 11,26 (16 %) und 11,49 min (50 %). Enantiomerenüberschuß 52 %.

Eine racemische Probe ergab ein 1;1-Diastereomerengemisch.

### Beispiel 8 (nicht erfindungsgemäß)

a) Eine Lösung von 5,00 g (S)-2-(tert. Butoxycarbonylamino-1-phenyl-but-3-en [hergestellt nach J. Org. Chem. 52,1487 (1987)] in 100 ml einer 4 N-Lösung von gasförmigem Chlorwasserstoff in wasserfreiem Dioxan wurde 30 min bei 20°C gerührt. Danach wurden 15 ml Toluol zugegeben und im Vakuum eingeengt. Die Toluolzugabe und das Einengen wurden noch zweimal wiederholt, dann der Rückstand mit wenig Ether verrieben, abgesaugt und im Hochvakuum über Kaliumhydroxid getrocknet. Es wurden 3,69 g (= 99 % der Theorie) (S)-2-Amino-1-phenyl-but-3-en-hydrochlorid erhalten.
b) Eine auf 0°C gekühlte, gerührte Lösung von 4,81 g N-(tert. Butylcarbonyl)-L-valin und 3,29 g 1-Hydroxybenzotriazol in 40 ml wasserfreiem Dichlormethan wurde mit 5,29 g Dicyclohexylcarbodiimid versetzt und 5 min nachgerührt. Danach wurde eine Lösung von 3,70 g der gemäß a) erhaltenen Verbindung und 8,85 ml N-Methylmorpholin in 30 ml Dichlormethan zugetropft, die Kühlung entfernt und 2 Std. bei 20°C nachgerührt. Das Ende der Reaktion wurde durch Dünnschichtchromatographie überprüft. Der entstandene Harnstoff wurde durch Filtration abgetrennt, das Filtrat im Vakuum eingeengt und das so erhaltene Rohprodukt durch Chromatographie an 450 g Kieselgel (Dichlormethan: Methanol 95 : 5) gereinigt. Es wurden 6,07 g (= 0 87 % der Theorie) (2S)-2-[N-(tert. Butoxycarbonyl-L-valinyl)]-amino-1-phenylbut-3-en erhalten.
c) Aus 6,08 g der gemäß b) erhaltenen Verbindung wurden analog zu a) 4,90 g (= 99 % der Theorie) (2S)-1-Phenyl-2-(N-L-valinyl)-aminobut-3-en-hydrochlorid erhalten.

### Beispiel 9 (nicht erfindungsgemäß)

Eine auf 0°C gekühlte und gerührte Lösung von 1,50 g (4,47 mmol) (2S)-3-tert.-Butylsulfonyl-2-(1-naphthylmethyl)-propionsäure [hergestellt nach J. Med. Chem, 31, 1839 (1988)] und 0,66 g (4,92 mmol) 1-Hydroxybenzotriazol in 15 ml wasserfreiem Dichlormethan wurde mit 0,97 g (4,69 mmol) Dicyclohexylcarbodiimid versetzt und 5 min gerührt. Danach wurde eine Lösung von 1,15 g (4,07 mmol) (2S)-1-Phenyl-2-(N-L-valinyl)-aminobut-3-en-hydrochlorid und 1,80 ml (16,27 mmol) N-Methylmorpholin in 10 ml Dichlormethan zugetropft und 1 Std. bei Raumtemperatur gerührt. Man trennte den entstandenen Harnstoff durch Filtration ab, engte das Filtrat im Vakuum ein und reinigte das Rohprodukt durch Chromatographie an 270 g Kieselgel (Dichlormethan:Methanol 95 : 5). Man erhielt 2,01 g (= 88 % der Theorie) (2S)-2-[N-(2S)-3-(tert.-Butylsulfonyl)-2-(1-naphthylmethyl)-propanoyl]-L-valinyl]-amino-1-phenylbut-3-en der Formel 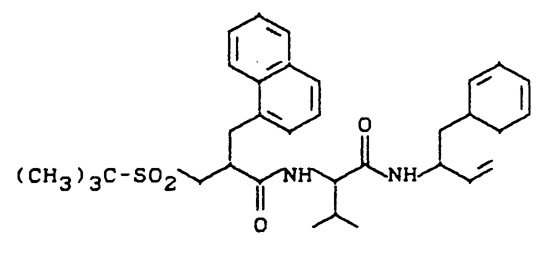
als farblosen Schaum.

### Beispiel 10 (nicht erfindungsgemäß)

Eine Suspension aus 0,67 mmol der Verbindung hergestellt nach Beispiel 9, 8 mg Benzyltriethylammoniumchlorid und 668 mg Magnesiummonoperoxiphthalat-hexahydrat in 3 ml Chloroform wurde durch Zugabe von 1N Natronlauge auf pH 5 eingestellt und 16 Std. am Rückfluß erhitzt, wobei durch weitere Zugabe von kleinen Mengen 1N Natronlauge der pH bei 5 gehalten wurde. Nach dem Abkühlen wurde das Reaktionsgemisch abgesaugt, das Filtrat nacheinander mit 10 ml Wasser, 10 ml 10 %iger Na₂SO₃-Lösung und 10 ml verdünnter NaHCO₃-Lösung gewaschen und über Magnesiumsulfat getrocknet. Nach dem Abdampfen des Lösungsmittels im Vakuum und Chromatographie des Rückstandes an 15 g Kieselgel (Toluol:Ethylacetat 1 : 1) wurde in 38 %iger Ausbeute das Epoxid der nach Beispiel 9 hergestellten Verbindung erhalten.

### Beispiel 11 (nicht erfindungsgemäß)

Eine Lösung von 15,18 mmol des gemäß Beispiel 10 erhaltenen Epoxids und 18,2 mmol der gemäß Beispiel 2 erhaltenen 1-(Trifluormethyl)-decahydroisochinolins in 4 ml Propanol wurde in einem Druckgefäß 2 Stunden lang bei 110°C gerührt. Nach dem Abkühlen wurde das Reaktionsgemisch im Vakuum eingeengt und nach Vorreinigung an 100 g Kieselgel an 600 g Kieselgel (Toluol:Ethylacetat 9:1) chromatographisch aufgetrennt. Man erhielt in 22 %iger Ausbeute die Verbindung der Formel 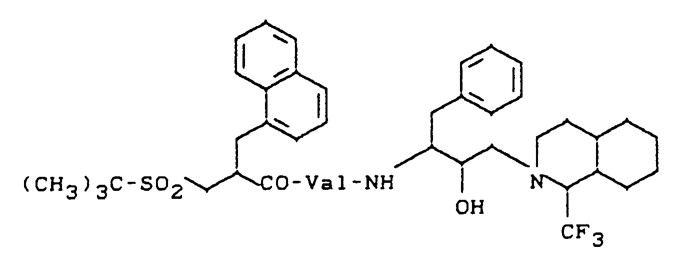
R_{f} 0,36 (Toluol/Ethylacetat 3:2) MS (FAB) m/z 786 (M+H⁺)

### Beispiel 12

Es wurde verfahren wie in Beispiel 11, jedoch wurden statt des gemäß Beispiel 2 erhaltenen 1-(Trifluormethyl)-decahydroisochinolins 18,2 mmol des gemäß Beispiel 3 erhaltenen 3-(Trifluormethyl)-decahydroisochinolins eingesetzt. Man erhielt in 14 %iger Ausbeute die Verbindung der Formel 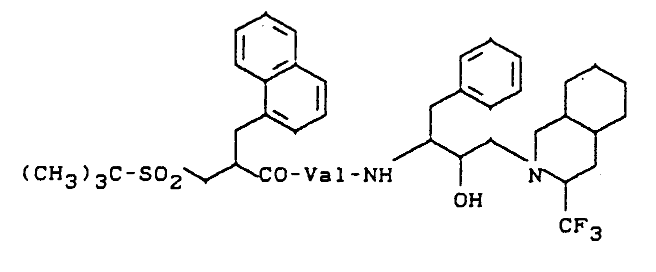
R_{f} 0,19 (Toluol/Ethylacetat 3:2)
MS (FAB) m/z 786 (M+H⁺)

### Beispiel 13

HIV-spezifischer Protease-Enzymtest entsprechend J. Hansen, S. Billich, T. Schulze, S. Sukrow und K. Mölling, EMBO-Journal, Vol. 7, Nr. 6, Seiten 1705-1791 (1988).

Dazu wurde gereinigte HIV-Protease mit synthetischem Peptid inkubiert, das eine Schnittstelle im Gag-Precursor-Protein imitiert und eine invivo-Spaltstelle der HIV-Protease darstellt. Die entstandenen Spaltprodukte des synthetischen Peptids wurden über Reverse High Performance Liquid Chromatography (RP-HPLC) analysiert. Mit der gemäß Beispiel 11 erhaltenen Verbindung wurde ein IC₅₀-Wert von 5 x 10⁻⁸ bei HIV-1 erhalten. IC₅₀ gibt dabei die Substanzkonzentration an, die unter den Testbedingungen eine 50 %ige Hemmung der Protease-Aktivität bewirkte.

### Beispiel 14

HIV-Test in Zellkultur mit geringen Modifikationen entsprechend Pauwels et al., Journal of Virological Methods 20, Seiten 309-321 (1988).

Normale menschliche Blutlymphozyten (PBL's) wurden über Ficoll-Hypaque angereichert und im RPMI 1640, 20 % fötales Kälberserum mit Phythaemagglutinin (90 µg/ml) und Interleukin-2 (40 U/ml) stimuliert. Zur Infektion mit dem infektiösen HIV wurde PBL's pelletiert und das Zellpellet wurde anschließend in 1 ml HIV-Virusadsorptionslösung suspendiert und 1 Stunde bei 37°C inkubiert.

Die Virusadsorptionslösung wurde zentrifugiert und das infizierte Zellpellet in Wachstumsmedium aufgenommen, so daß 1 x 10⁵ Zellen pro ml eingestellt waren. Die derart infizierten Zellen wurden zu 1 x 10⁴ Zellen/Napf in die Näpfe von 96 er Mikrotiterplatten pipettiert.

Die erste vertikale Reihe der Mirkotiterplatte enthielt nur Wachstumsmedium und Zellen, die nicht infiziert, aber ansonsten genauso wie oben beschrieben, behandelt worden waren (Zellkontrolle). Die zweite vertikale Reihe der Mikrotiterplatte enthielt nur HIV-infizierte Zellen (Viruskontrolle) in Wachstumsmedium. Die übrigen Näpfe enthielten die gemäß Beispiel 11 erhaltene Verbindung in unterschiedlicher Konzentration, ausgehend von den Näpfen der 3. vertikalen Reihe der Mikrotiterplatte, von der die Prüfsubstanz in 2er Schritten 2¹⁰fach verdünnt wurde.

Die Testansätze wurden so lange bei 37°C inkubiert, bis in der unbehandelten Viruskontrolle die für das HIV typische Syncytienbildung auftrat (zwischen Tag 3 und 6 nach Infektion), die dann mikroskopisch ausgewertet wurde. In der unbehandelten Viruskontrolle resultierten unter diesen Testbedingungen etwa 20 Syncytien, während die unbehandelten Zellkontrolle keine Syncytien aufwies.

Der IC₅₀-Wert wurde als die Konzentration der behandelten und infizierten Zellen ermittelt, bei der 50 % (ca. 10 Syncytien) der virusinduzierten Syncytien durch die Behandlung mit der gemäß Beispiel 11 erhaltenen Verbindung unterdrückt waren.

Es wurde nun gefunden daß diese Verbindungen HIV infizierte Zellen vor der virusinduzierten Zellzerstörung schützt.

Als IC₅₀-Wert (PBL) wurde 2,5 µM ermittelt.

## Patentansprüche

1. α-Trifluormethyl-substituierte, gesättigt-bicyclische Amine der Formel (I) 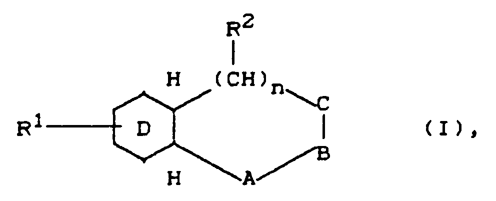 in der
R¹ für Wasserstoff, C₁- bis C₈-Alkyl oder C₁- bis C₈- Alkoxy,
R² für Wasserstoff oder C₁- bis C₄-Alkyl,
n für Null oder 1,
A für 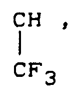 NH oder 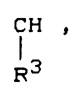
B für NH oder 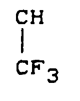 und
C für 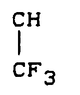 oder 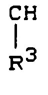 stehen,
wobei A, B und C entweder jeweils verschieden voneinander sind oder C für 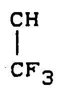 und eines von A und B für NH und das andere für 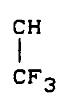 steht
und R³ Wasserstoff oder C₁- bis C₄-Alkyl bedeutet und
der Ring D gesättigt ist.

2. Amine gemäß Anspruch 1, dadurch gekennzeichnet, daß in Formel (I)
R¹ für Wasserstoff, Methyl oder Methoxy,
R² für Wasserstoff und/oder
R³ für Wasserstoff stehen.

3. Verfahren zur Herstellung von Aminen der Ansprüche 1 und 2, dadurch gekennzeichnet, daß man entsprechende ungesättigte Verbindungen mit molekularem Wasserstoff in Gegenwart eines Ruthenium-Katalysators hydriert.

4. Verfahren nach Anspruch 3, dadurch gekennzeichnet, daß man nicht- oder teilhydrierte Chinoline, Isochinoline oder Indole hydriert, die α-ständig zum Stickstoffatom einen Trifluormethyl-Substituenten, am Stickstoff enthaltenen Ring gegebenenfalls 1 bis 2 C₁- bis C₄-Alkyl-Substituenten und am carbocyclischen Ring gegebenenfalls einen C₁-bis C₈-Alkyl- oder C₁-bis C₈- Alkoxy-Substituenten enthalten.

5. Verfahren nach Ansprüchen 3 und 4, dadurch gekennzeichnet, daß man die Hydrierung bei 20 bis 150°C und 60 bis 180 bar durchführt.

6. Verfahren nach Ansprüchen 3 bis 5, dadurch gekennzeichnet, daß man die Hydrierung bei 70 bis 110°C und 80 bis 120 bar durchführt.

7. Verfahren nach Ansprüchen 3 bis 6, dadurch gekennzeichnet, daß man einen Trägerkatalysator einsetzt, der 0,1 bis 20 Gew.-% Ruthenium und/oder Rutheniumverbindungen enthält.
